# FASCICULE DE BREVET EUROPEEN

(11) **EP 1 307 245 B1**
(45) Date de publication et mention de la délivrance du brevet: **19.10.2005**
(21) Numéro de dépôt: 01960870.2
(22) Date de dépôt: 06.08.2001
(51) Int. Cl.: A61L 24/04, A61P 17/02, A61P 25/00

(54) **FORME PHARMACEUTIQUE COMPRENANT UN FACTEUR DE REGULATION CELLULAIRE ET/OU UN PROMOTEUR DE PROLIFERATION CELLULAIRE**
ARZNEIMITTELFORMEN MIT ZELLREGULIERENDEM FAKTOR UND/ODER EINEM PROMOTER ZUR PROLIFERATION VON ZELLEN
PHARMACEUTICAL FORM COMPRISING A CELL REGULATING FACTOR AND/OR A CELL PROLIFERATION PROMOTER

(30) Priorité: 07.08.2000 FR 0010396
(43) Date de publication de la demande: 07.05.2003
(73) Titulaire: Halisol, 75116 Paris (FR)
(72) Inventeur: BRU-MAGNIEZ, Nicole, F-75016 Paris (FR); MYSIAKINE, Evgueni, F-75015 Paris (FR); FATTAL, Elias, F-75012 Paris (FR); COUVREUR, Patrick, F-91140 Villebon sur Yvette (FR); BRETON, Pascal, F-45510 Tigy (FR)
(74) Mandataire: Hubert, Philippe
(86) Numéro de dépôt international: PCT/FR2001/002554
(87) Numéro de publication internationale: WO 2002/011779

(56) Documents cités:
- EP-A- 0 727 230
- FR-A- 2 755 136
- FR-A- 2 778 100
- FR-A- 2 789 314
- US-A- 5 374 616
- US-A- 5 707 647

## Description

L'invention concerne une nouvelle forme pharmaceutique comprenant un matériau support et au moins un facteur de régulation cellulaire et/ou un promoteur de prolifération cellulaire.

Dans la présente description, on entend par le terme « matériau support » un matériau polymérique biocompatible susceptible de comporter, à sa surface ou au sein de sa structure, au moins un facteur de régulation cellulaire et/ou un promoteur de prolifération cellulaire.

On entend par « facteurs de régulation cellulaire » des médiateurs biochimiques sécrétés par les cellules et susceptibles d'intervenir dans des mécanismes cellulaires tels que par exemple le développement, la croissance ou l'activation des cellules, en particulier les cytokines, les chimiokines et les facteurs de croissance, ces derniers étant particulièrement préférés. Lesdits facteurs de régulation cellulaire peuvent être naturels, synthétiques ou recombinants.

Par « promoteur de prolifération cellulaire », on entend une molécule naturelle, recombinante ou synthétique modulant les voies de signalisation des facteurs de régulation cellulaire et ayant un effet activateur de la prolifération cellulaire, telle que par exemple la sphingosylphosphorylcholine .

L'invention trouve une application intéressante dans le domaine du traitement des lésions cutanées ou des plaies dermiques et dermoépidermiques, qui nécessitent la favorisation de la croissance des cellules impliquées dans le processus de cicatrisation, par exemple les brûlures, en particulier les brûlures des second et troisième degrés, les ulcères, les escarres, les ulcères cutanés ou variqueux.

Elle trouve également une application intéressante dans une utilisation en tant qu'implant dans laquelle le matériau support, biodégradable, est susceptible de libérer progressivement le ou les facteur(s) de régulation cellulaire, en particulier le ou les facteur(s) de croissance, et /ou le ou les promoteur(s) de prolifération cellulaire au niveau du site d'implantation.

La réparation des lésions cutanées met en jeu une série complexe de processus cellulaires comprenant la contraction de la blessure, l'accumulation locale de cellules inflammatoires, la néovascularisation, l'activation et la prolifération de cellules stromales (fibroblastes, histiocytes etc.), la synthèse de composants de la matrice extracellulaire (collagène etc.) et l'épithélialisation.

*In vivo,* ces processus sont régulés par différentes cytokines, chimiokines, et eicosanoïdes.

Parmi ceux-ci, la famille des facteurs de croissance des fibroblastes, communément dénommés FGF (pour « fibroblast growth factor »), dont on connaît actuellement 22 formes qui diffèrent par leur spécificité vis-à-vis de leurs cellules cibles, et dont certaines, comme le FGF-1 ou encore le FGF-2 présentent l'avantage d'être susceptibles de réguler la croissance de tous les types de cellules impliqués dans la cicatrisation des plaies.

Une autre famille intéressante est celle des facteurs de croissance nerveux ou NGF (pour « Nerve Growth Factor ») qui peuvent être impliqués dans la régénération des tissus nerveux, neuromusculaires et neuroépithéliaux.

Il est donc d'un grand intérêt de disposer d'une forme pharmaceutique permettant leur apport et leur maintien au niveau de la lésion à traiter, tout en préservant leur activité stimulatrice de la croissance cellulaire.

Le document EP 0 727 230 décrit généralement un adhésif biocompatible destiné à la fixation d'un dispositif chirurgical implantable réalisé en un matériau polymérique qui peut être notamment un poly dialkylméthylène malonate.

Le document US 5,707,647 révèle l'utilisation d'une matrice polymérique, par exemple à base de polylactide, de polycaprolactone ou de polyglycolide incorporant un facteur de croissance pour la réalisation d'un revêtement d'un implant.

Le document FR 2 755 136 décrit généralement un procédé de fabrication de nanoparticules à base de poly méthylidène malonate et leur utilisation comme véhicule pour des molécules biologiquement actives comme des agents anti-infectieux.

Le document FR 2 778 100 décrit un procédé de préparation de microsphères à base de polyméthylidène malonate et leur utilisation pour l'encapsulation de molécules biologiquement actives telles que des agents anti-infectieux ou lovalbumines.

Dans ce contexte, l'invention a donc pour objet une forme pharmaceutique comprenant un matériau support et au moins un facteur de régulation cellulaire et/ou un promoteur de prolifération cellulaire, caractérisé en ce que le matériau support est constitué, pour au moins 90 % en poids et de préférence pour au moins 95 % en poids, d'une composition à base de méthylidène malonate contenant :
- de 0 à 10 % en poids d'un ou plusieurs méthylidène malonate de formule (I) 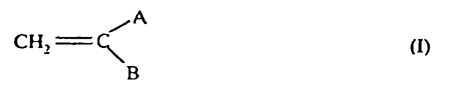
dans laquelle :
- A et B représentent indépendamment un groupe (a) ou (b) : 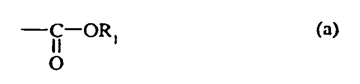 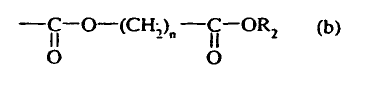
dans lesquels R₁ et R₂ représentent indépendamment un groupe alkyle linéaire ou ramifié ayant de 1 à 6 atomes de carbone, et n est un nombre entier compris entre 1 et 5 ;
- de 10 à 90 %, de préférence 50 à 90 % en poids d'un ou plusieurs oligomère(s) de méthylidène malonate présentant un poids moléculaire inférieur ou égal à 6 000 et constitué(s) d'unités récurrentes de formule (II) : 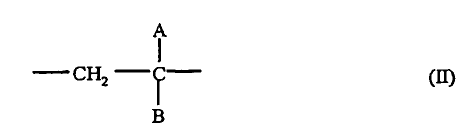
dans laquelle A et B sont tels que définis précédemment ;
- de 10 à 90 %, de préférence 10 à 50 % en poids d'un ou plusieurs polymère(s) de méthylidène malonate présentant un poids moléculaire supérieur à 6 000 et constitué(s) d'unités récurrentes de formule (II).

Par « poids moléculaire supérieur à 6000 », on entend notamment un poids moléculaire supérieur à 9 000, en particulier supérieur à 12 000, notamment supérieur à 25 000, de préférence supérieur à 50 000, voire allant jusqu'à 600 000.

Dans la description, on entend par « poids moléculaire » la masse molaire moyenne en poids désignée M_{w}, exprimée en g/mole d'équivalent polystyrène (PS), et mesurée par Chromatographie par Perméation de Gel (CPG) avec un appareillage chromatographique étalonné avec des polymères étalons de polystyrène.

Selon un aspect préféré, dans les formules (I) et (II) précitées :
- A représente un groupe (a) dans lequel R₁ représente un groupe éthyle ; et
- B représente un groupe (b) dans lequel R₂ représente un groupe éthyle et n est un nombre égal à 1.

Dans un mode de réalisation préféré, le matériau support est constitué pour au moins 90%, et de préférence pour au moins 95% en poids d'une composition à base de méthylidène malonate contenant :
- de 50 à 90 % en poids d'un ou plusieurs oligomère(s) de méthylidène malonate présentant un poids moléculaire inférieur ou égal à 6 000, de préférence inférieur ou égal à 3 000, et constitué(s) d'unités récurrentes de formule (II),
- de 10 à 50 % en poids d'un ou plusieurs polymères de méthylidène malonate présentant un poids moléculaire supérieur à 6 000.

Selon un aspect avantageux, la composition à base de méthylidène malonate contient :
- de 55 à 65 % en poids d'un ou plusieurs oligomère(s) de méthylidène malonate présentant un poids moléculaire inférieur ou égal à 3 000, notamment compris entre 300 et 1 000 et constitué(s) d'unités récurrentes de formule (II),
- de 35 à 45 % en poids d'un ou plusieurs polymères de méthylidène malonate présentant un poids moléculaire supérieur à 6 000, de préférence supérieur à 9 000, et plus particulièrement compris entre 12 000 et 25 000.

D'autres compositions avantageuses à base de méthylidène malonate contiennent :
- de 40 à 80 %, en particulier de 40 à 60 % en poids d'un ou plusieurs oligomère(s) de méthylidène malonate présentant un poids moléculaire inférieur ou égal à 6 000, de préférence inférieur ou égal à 3 000, et constitué(s) d'unités récurrentes de formule (II),
- de 20 à 60 %, en particulier de 40 à 60 % en poids d'un ou plusieurs polymères de méthylidène malonate présentant un poids moléculaire supérieur à 6 000, de préférence supérieur à 9 000, et plus particulièrement compris entre 12 000 et 25 000.

Selon un aspect préféré de l'invention, le facteur de régulation cellulaire utilisable aux fins de l'invention est un facteur de croissance. Avantageusement, le ou les facteur(s) de croissance, peut(vent) être par exemple choisis(s) parmi les facteurs de croissance des fibroblastes (FGF), le « Transforming Growth Factor β »(TGFβ), le « Platelet-Derived Growth Factor » (PDGF), le « Nerve Growth Factor » (NGF) ou une association de plusieurs d'entre eux.

Selon un aspect avantageux, le ou les facteur(s) de croissance est (sont) utilisé(s) seul(s) ou en complexe avec un ligand. En effet, la famille des facteurs de croissance des fibroblastes forment avec leurs ligands naturels habituellement présents dans les tissus tels que l'héparine, l'héparane sulfate ou les protéoglycanes d'héparane sulfate, des complexes très stables dans les conditions physiologiques.

De préférence, on utilisera le facteur de croissance des fibroblastes basique, également dénommé FGF-2, en complexe avec l'héparane sulfate ou avec un protéoglycane d'héparane sulfate.

Le FGF-2 et l'héparane sulfate peuvent notamment être présents au sein dudit complexe dans un rapport molaire de 1 : 1 à 1 : 20, en particulier de 1 : 1,5 à 1:10.

Dans le cas d'un complexe avec un protéoglycane d'héparane sulfate, le FGF-2 et le protéoglycane d'héparane sulfate peuvent notamment être présents au sein dudit complexe dans un rapport molaire de 1 : 0,01 à 1 : 0,5.

La préparation de la forme pharmaceutique selon l'invention comprend l'incorporation du ou de(s) facteur(s) de régulation cellulaire ou du ou des promoteur(s) de prolifération cellulaire, en particulier du ou de(s) facteur(s) de croissance, seul(s) ou sous forme de complexe(s) avec un ligand, dans le matériau support.

Le matériau support peut être préparé par un procédé dans lequel le mélange souhaité de monomère(s) et/ou d'oligomère(s) et/ou de polymère(s) est réalisé *in situ* en modulant les conditions de réaction.

Alternativement, le matériau support est préparé par mélange de différentes quantités de monomère(s) et/ou d'oligomère(s) et/ou de polymère(s), lesdits oligomère(s) et polymère(s) étant préalablement préparés, afin d'obtenir la proportion souhaitée de chacun des constituants.

Une solution aqueuse du ou des facteur(s) de régulation cellulaire et/ou du ou des promoteur(s) de prolifération cellulaire est ensuite incorporée au matériau support.

Alternativement, lorsque le matériau support est obtenu par mélange de différentes quantités de monomère(s) et/ou d'oligomère(s) et/ou de polymère(s) préalablement préparés, la solution aqueuse du ou des facteur(s) de régulation cellulaire et/ou du ou des promoteur(s) de prolifération cellulaire peut être incorporée au matériau support durant l'une des étapes de préparation dudit matériau support.

Dans les deux cas, cette incorporation est effectuée de telle manière que le ou les facteur(s) de régulation cellulaire et/ou le ou les promoteur(s) de prolifération cellulaire ne subisse(nt) aucune dénaturation, et soit(soient) présent(s) à la fois à la surface et /ou dans la structure dudit matériau support.

Le matériau support conforme à la présente invention est essentiellement caractérisé par le fait qu'il est constitué majoritairement d'une composition à base de méthylidène malonate, elle-même majoritairement constituée de monomère(s) et/ou d'oligomère(s) de poids moléculaire inférieur ou égal à 6000, de préférence inférieur ou égal à 3000.

Selon un autre aspect avantageux de l'invention, cette composition est constituée majoritairement d'oligomère(s) de poids moléculaire inférieur ou égal à 6000 et de polymère(s) de poids moléculaire supérieur ou égal à 6000.

De telles compositions présentent des propriétés de viscosité et de bioadhésion permettant leur utilisation dans le traitement des plaies dermiques et dermoépidermiques, seule ou en mélange avec d'autres composants biocompatibles, notamment pour une utilisation de la forme pharmaceutique selon l'invention en tant qu'implant.

En outre, les compositions à base de méthylidène malonate utilisables dans le cadre de l'invention sont biodégradables, en libérant de l'éthanol et de l'acide glycolique qui sont généralement considérés comme non toxiques pour l'organisme aux doses utilisées. Cette biodégradabilité présente un grand intérêt pour la libération du ou des facteur(s) de croissance contenu(s) notamment dans la structure oligomérique ou polymérique du matériau support au niveau du site de la lésion à traiter.

De plus, les compositions à base de méthylidène malonate permettent avantageusement de stabiliser le facteur de régulation cellulaire ou promoteur de prolifération cellulaire dans le milieu dans lequel il est utilisé, cette stabilité étant notamment mesurable par le maintien de leur activité biologique dans le temps par rapport à celle d'un tel facteur libre dans le milieu.

Ces compositions à base de méthylidène malonate pourront être facilement préparées par l'homme de métier, éventuellement par simple mélange dans un solvant approprié de ses constituants (monomériques, oligomériques, polymériques) préparés séparément, et évaporation subséquente du solvant.

Ainsi, les monomères de méthylidène malonate pourront être préparés en suivant le procédé décrit dans le brevet EP 0 283 346 correspondant aux brevets US 4 931 584 et US 5 142 098, après dégazage sous vide de pompe à palettes jusqu'à poids constant pour éliminer l'inhibiteur de polymérisation (SO₂).

Les oligomères et polymères de méthylidène malonate pourront être synthétisés par voie anionique ou radicalaire à partir des monomères précités.

Dans le cas des compositions à base de méthylidène malonate préférées, qui sont formées d'un mélange d'oligomère(s) et de polymère(s), ces compositions pourront être obtenues également en une seule étape; les proportions relatives des constituants pourront être ajustées en faisant varier la concentration en amorceur anionique ou radicalaire dans le milieu de polymérisation.

Les caractéristiques physico-chimiques des compositions à base de méthylidène malonate précitées pourront ainsi être facilement ajustées par l'homme de métier, pour obtenir le matériau support selon l'invention.

D'une façon générale, les constituants du matériau de support conforme à l'invention, autres que la composition à base de méthylidène malonate précitée, pourront représenter jusqu'à 10 % en poids de ce matériau.

Ces constituants seront bien entendu choisis de manière à former des mélanges intimes avec les compositions à base de méthylidène malonates précitées.

Ces constituants pourront être de nature variée, hydrophobes ou hydrophiles, d'origine naturelle ou synthétique.

A titre d'exemple de tels constituants, on citera notamment :
- les polycyanoacrylates, de préférence les polyalkylcyanoacrylates ;
- les polyméthacrylates d'alkyle ;
- les polyuréthanes biocompatibles ;
- les polyoxyalkylènes ;
- les polyaminoacides ;
- les polyalcoolvinyliques.

D'une façon générale, ces constituants seront présents au sein du matériau support sous forme de mélanges avec les compositions à base de méthylidène malonate précitées.

Il convient de noter que, sans sortir du cadre de la présente invention, ces constituants pourront également être présents au sein du matériau support sous forme d'unités monomériques dans des copolymères statistiques, multiblocs ou greffés comprenant des unités méthylidène malonate de formule (II), telle que définie précédemment.

Ces copolymères à base de méthylidène malonate pourront être préparés par les techniques de polymérisation classiques bien connues de l'homme de métier, parmi lesquelles on citera la polymérisation par voie anionique, la polymérisation par voie radicalaire ou encore, dans le cas des copolymères mutiblocs ou greffés, la technique de couplage des séquences précurseurs du copolymère, ces séquences ayant été au préalable fonctionnalisées en bout de chaîne de façon adéquate.

Ces constituants autres que le méthylidène malonate peuvent également être présents au sein du matériau support sous forme d'unités monomériques enchaînées pour former des homopolymères ou des copolymères statistiques, multiblocs ou greffés dépourvus d'unités méthylidène malonate de formule (II).

D'une façon générale, les unités monomériques formant les constituants précités seront choisies parmi les unités monomériques constitutives des polyacrylates, des polysaccharides et des polyoxyalkylènes.

Parmi les unités monomériques constitutives de polyacrylates susceptibles d'être utilisées dans le cadre de l'invention, on citera les alkylcyanoacrylates, les méthacrylates d'alkyle et les itaconates.

D'une façon générale, les copolymères à base de méthylidène malonate utilisés dans le cadre de la présente invention seront constitués, pour au moins 50 % de leurs unités monomériques, par des unités méthylidène malonate.

Selon un aspect ultérieur, l'invention concerne une composition pharmaceutique comprenant une forme pharmaceutique telle que décrite ci-dessus et un excipient pharmaceutiquement acceptable.

La forme pharmaceutique selon l'invention ou la composition pharmaceutique la contenant peut être administrée par voie topique ou injectable.

L'invention concerne également l'utilisation d'une forme pharmaceutique telle que définie plus haut pour la fabrication d'un implant ou pour la préparation d'une composition destinée à être placée au contact d'une plaie ou encore pour la préparation d'un médicament pour le traitement des lésions cutanées ou des plaies dermiques ou dermoépidermiques, par exemple les brûlures en particulier les brûlures des second et troisième degrés, les ulcères, les escarres, les ulcères cutanés ou variqueux.

Elle concerne également l'utilisation d'une forme pharmaceutique telle que définie plus haut pour la préparation d'un médicament pour la régénération des tissus nerveux, neuromusculaires ou neuroépithéliaux.

Selon un aspect ultérieur, l'invention a pour objet un procédé de traitement des lésions cutanées et des plaies dermiques ou dermoépidermiques, en particulier les brûlures, caractérisé en ce qu'il consiste à appliquer sur ladite lésion ou plaie une quantité efficace de la forme pharmaceutique telle que définie plus haut.

Les exemples suivants illustrent l'invention de manière non limitative.

### Exemple 1 : Préparation d'une forme pharmaceutique selon l'invention contenant un complexe FGF-2/ héparane sulfate

Dans une fiole de 25 ml, on introduit 40 mg de monomère de méthylidène malonate (1-éthoxycarbonyl-1-éthoxycarbonylméthylènoxycarbonyl éthène), maintenu sous vide primaire afin d'éliminer l'inhibiteur de polymérisation (dioxyde de soufre) et dissout dans 2 ml d'acétone. L'agitation magnétique est maintenue pendant 15 min.

On ajoute ensuite 0,4 ml de NaOH 0,1N en une seule étape toujours sous agitation magnétique.

L'agitation magnétique est maintenue pendant 20 min environ. On obtient un volume de 2,4 ml contenant 40 mg de matériau support. Après évaporation sous vide de l'acétone, ledit matériau est amené au 1/5 de son volume.

On obtient ainsi un matériau support constitué d'une composition de méthylidène malonate contenant environ 90 % d'oligomères de poids moléculaire environ égal à 3000 et 10 % de polymère de poids moléculaire compris entre environ 20 000 et 25 000.

On ajoute à température ambiante un volume égal d'une solution aqueuse contenant un complexe FGF-2/héparane sulfate (dénommé ci-après FGF-2/HS).

Ce complexe est préalablement préparé en mélangeant les 2 composants dans 100 µl de tampon Tris 5 mM pH 7,6, le mélange étant laissé sous agitation magnétique pendant 2 h à température ambiante.

Les différents complexes indiqués dans le tableau 1 ci-dessous ont été préparés en utilisant du FGF-2 recombinant humain (TEBU, France) et de l'héparane sulfate (SIGMA) purifié par électrophorèse, fraction de poids moléculaire 7,5 kD .

**TABLEAU 1**

| No. Complexe | µg FGF-2 | µg héparane sulfate | rapport molaire |
|---|---|---|---|
| 1 | 1 | 0,8 | 1 : 1,5 |
| 2 | 1 | 3,4 | 1 : 6,3 |
| 3 | 1 | 5,3 | 1 : 10 |
| 4 | 1 | 10,7 | 1 : 20 |

Après l'addition du complexe FGF-2/héparane sulfate, le mélange est soumis à une évaporation rapide jusqu'à l'obtention d'un volume de 0,5 à 0,7 ml, puis à une évaporation sous vide pendant 1 nuit en étuve jusqu'à séchage complet.

On obtient ainsi 40 mg de forme pharmaceutique selon l'invention.

### Exemple 2 : Evaluation de l'activité stimulatrice de la prolifération cellulaire des formes pharmaceutiques contenant des complexes FGF-2/HS

Les formes pharmaceutiques selon l'invention contenant les complexes FGF-2/HS de l'exemple 1 ont été mises en contact avec un milieu conditionné, c'est-à-dire un milieu de culture dans lequel les cellules ont été cultivées pendant 2-3 jours et qui contient donc des enzymes secrétées par ces cellules, provenant d'une culture d'entérocytes humains Caco-2 (n°ATCC HTB-37).

Les formes pharmaceutiques sont mises en contact avec ce milieu à raison de 40 mg de forme pharmaceutique par ml de milieu à 37°C pendant 24h. Après centrifugation à 500 g pendant 10 min, le surnageant est introduit dans le milieu de culture des cellules BP-A31 quiescentes, à raison de 2,5% v/v (5µl pour 200µl).

On a utilisé la lignée cellulaire de fibroblastes de souris BP-A31 (fibroblastes 3T3 chimiquement transformés exprimant une haute teneur en récepteurs au FGF - T.Buchou et al., Exp. Cell. Res ., 1988, 174(2), 411-420).

Les cellules sont cultivées pendant 24 h dans des plaques à 24 puits à raison de 40 000 cellules par puits (ou 8 000 cellules par puits dans des plaques à 96 puits) dans du milieu DMEM (GIBCO) contenant 8 % de sérum de veau foetal et des antibiotiques.

Elles sont ensuite cultivées pendant 48 h en milieu dépourvu de sérum afin d'obtenir une culture quiescente et d'augmenter leur sensibilité au FGF exogène, avant l'addition des échantillons à tester, dans le même milieu.

Après incubation pendant 24 h à 37°C, le taux de prolifération est estimé en utilisant le test MTT(T.Mosmann, J.Immunol.Meth.,1983, 65, 55-63).

Le MTT (bromure de diméthylthiazol phényl tétrazolium) est un colorant jaune qui, au contact des cellules vivantes, est réduit en MTT formazan, par la chaîne respiratoire mitochondriale des cellules.

Le MTT formazan se présente sous forme de cristaux bleu-violet qui, une fois dissous, donnent une coloration proportionnelle au nombre de cellules vivantes.

Le mode opératoire utilisé pour la mesure est le suivant :
- on met 200 µl de la suspension cellulaire dans chaque puits (plaques de 96 puits NUNCLON DELTA)
- on incube à 37°C, air 95% CO2 5%, pendant un temps déterminé en fonction du protocole expérimental c'est-à-dire 24 h avec sérum, 48 h sans sérum, 24 h avec produits toujours sans sérum.
- on ajoute 20 µl d'une solution de MTT à 5 mg/ml dans chaque puits sans éliminer le milieu. La solution mère de MTT à 5 mg/ml est préparée dans du sérum physiologique et filtrée sur 0,22 µm pour stérilisation.
- on incube pendant 2h30 à 37°C, air 95% / CO2 5%
- le surnageant est éliminé par centrifugation à 500 g pendant 10 min puis on élimine le milieu par aspiration.
- on ajoute sur les cellules contenues dans chaque puits 200 µl de solution d'extraction préparée selon les proportions suivantes :

| | |
|---|---|
| 10g | SDS (lauryl sulfate de sodium) |
| 50ml | DMF (diméthyl formamide) |
| 50ml | Eau distillée |

Le pH est ajusté à 4,7 avec une goutte d'acide acétique.

Les boîtes sont agitées pendant 1 heure ou laissées à 37°C pendant une nuit, et on vérifie au microscope que tous les cristaux de méthyl formazan sont dissous.

La lecture est effectuée à 570 nm (lecteur de plaque ELISA Labsystems Multiscan MS), en utilisant des blancs de lecture contenant uniquement la solution d'extraction.

Les résultats exprimés en pourcentage de prolifération cellulaire pendant 24 h par rapport à une culture témoin sans addition de facteur de croissance, sont rapportés dans le tableau 2 ci-dessous :

**TABLEAU 2**

| Produit testé | % prolifération |
|---|---|
| forme pharmaceutique contenant le complexe n° 1 | 145 % |
| forme pharmaceutique contenant le complexe n° 2 | 210 % |
| forme pharmaceutique contenant le complexe n° 3 | 177% |
| forme pharmaceutique contenant le complexe n° 4 | 158 % |

Les résultats montrent que les formes pharmaceutiques selon l'invention stimulent la prolifération des cellules BP-A31.

### Exemple 3 : Préparation et évaluation de nouvelles formes pharmaceutiques contenant le complexe FGF-2/HS

Dans un tube à essai de 20 ml, on introduit 40 mg de matériau composé de différents pourcentages d'oligomères et de polymères de méthylidène malonate préparés à partir du monomère 1-éthoxycarbonyl-1-éthoxycarbonylméthylènoxycarbonyl éthène. Le matériau est dissous dans 1 ml d'acétone sous agitation constante pendant 15 minutes. Après évaporation sous vide de l'acétone, ledit matériau est ramené à 1/5 de son volume, soit 200 µl.

On ajoute à température ambiante un volume égal d'une solution aqueuse contenant un complexe FGF-2/HS. Ce complexe est préalablement préparé comme sus-indiqué (exemple 1), et le mélange est laissé sous agitation magnétique pendant 2 h à température ambiante. Après addition du complexe FGF-2/HS, le mélange est directement soumis à une évaporation sous vide pendant une nuit jusqu'à séchage complet. On obtient ainsi 40 mg de forme pharmaceutique.

Trois formes pharmaceutiques selon l'invention sont préparées variant en proportion d'oligomère/polymère, ce qui affecte les propriétés de viscosité et de bioadhésion: 40/60 (forme la plus solide), 60/40 et 80/20 (forme la plus liquide).

Ces formes pharmaceutiques variant en proportion oligomère/polymère selon l'invention et contenant ou non les complexes FGF-2/HS sont mises en contact avec les cellules BP-A31 suivant le protocole décrit dans l'exemple 1 afin de déterminer la toxicité des formes pharmaceutiques et l'activité biologique de stimulation de la prolifération par les formes pharmaceutiques contenant les complexes FGF-2/HS.

Les résultats donnés dans le Tableau 3 comparent l'effet sur la prolifération des diverses formes pharmaceutiques différant en proportion oligomère/polymère avec ou sans complexe FGF-2/HS. Les résultats correspondent à 3,1 % de formes pharmaceutiques dans le milieu. Ils sont exprimés en pourcentage de prolifération cellulaire pendant 24 h, par rapport à une culture témoin sans addition de matériau support.

**TABLEAU 3**

| rapport oligomère/polymère % | Prolifération (%) | |
|---|---|---|
| | sans FGF-2/HS | avec FGF-2/HS |
| 40/60 | 109,6 ± 11,5 | 198 ± 8,5 |
| 60/40 | 108,9 ± 9,3 | 201,6 ± 9,8 |
| 80/20 | 105,3 ± 12,6 | 194,7±13,2 |

Les résultats montrent que le FGF-2 formulé au sein des trois formes pharmaceutiques selon l'invention présente une activité stimulatrice de la prolifération cellulaire. De plus, en l'absence de complexe FGF-2/HS, le pourcentage de prolifération reste supérieur à 100%, ce qui montre que les trois matériaux supports ne présentent pas de toxicité détectable à la dose utilisée.

### Exemple 4 : Effet de la formulation du complexe FGF-2/HS dans les formes pharmaceutiques sur la stabilité de l'activité biologique du FGF-2.

On utilise d'une part les trois formes pharmaceutiques de l'exemple 3, dont le matériau support est constitué de différentes proportions pondérales d'oligomères et de polymères de méthylidène malonate et d'autre part du FGF-2 libre (non formulé). Après stockage à 4°C des 3 formes pharmaceutiques et du FGF-2 libre pendant 7, 14 et 21 jours, l'activité stimulatrice de la prolifération cellulaire est mesurée par le test MTT décrit dans l'exemple 2 pendant 24h par rapport à une culture témoin sans addition de facteur de croissance .

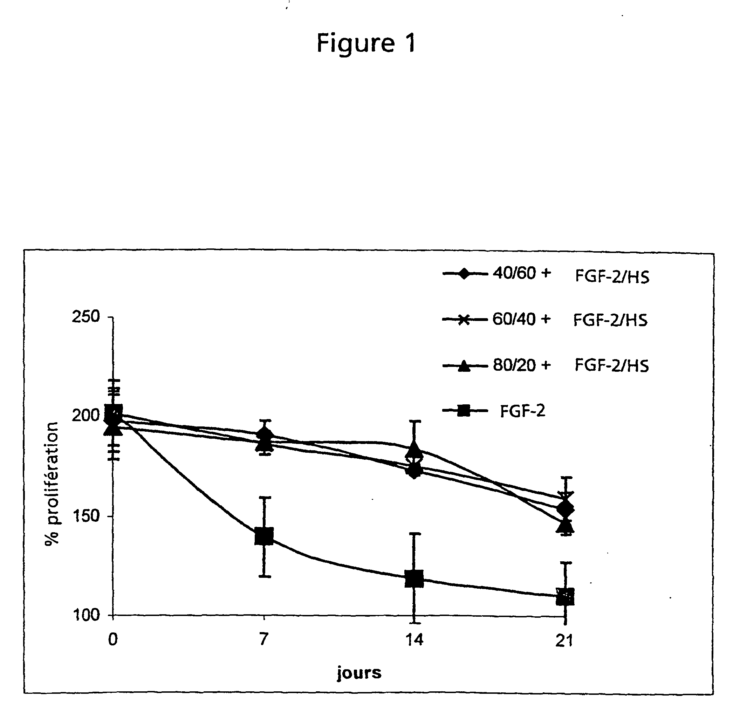

Les résultats sont représentés graphiquement sur la Figure 1, sur laquelle sont représentés en abscisse la durée de stockage à 4°C exprimée en jours et en ordonnée le pourcentage de prolifération.

Les symboles suivants sont utilisés :
-◆- représente la forme pharmaceutique comprenant un matériau support constitué de 40% d'oligomère et 60% de polymère et le complexe FGF-2/HS
-×- représente la forme pharmaceutique comprenant un matériau support constitué de 60% d'oligomère et 40% de polymère et le complexe FGF-2/HS
-▲- représente la forme pharmaceutique comprenant un matériau support constitué de 80% d'oligomère et 20% de polymère et le complexe FGF-2/HS
-■- représente le FGF-2 libre (non formulé)

Les résultats montrent que les trois formes pharmaceutiques maintiennent l'activité stimulatrice de la prolifération cellulaire du FGF-2 pendant une période d'au moins 21 jours. En revanche, le FGF-2 libre (non formulé) ne présente une activité comparable au FGF-2 formulé dans une forme pharmaceutique selon l'invention qu'au temps le plus précoce (zéro jour) et perd rapidement son activité biologique (dès 7 jours).

### Exemple 5 : Préparation et étude de la stabilité de formes pharmaceutiques contenant les facteurs de croissance PDGF et TGF-β

Le PDGF et le TGF-β ont été formulés au sein d'un matériau contenant, en poids, 60 % d'oligomère et 40 % de polymère de méthylidène malonate préparés à partir du monomère 1-éthoxycarbonyl-1-éthoxycarbonylméthylènoxycarbonyl éthène. On a utilisé 100 ng de PDGF ou de TGF-β pour 40 mg de matériau, comme décrit dans l'exemple 3. La forme pharmaceutique selon l'invention contenant ou non les facteurs de croissance a ensuite été mise en contact avec les cellules fibroblastiques L929 suivant le protocole décrit dans l'Exemple 1 et l'effet de stimulation de la prolifération du PDGF ou du TGF-β sur les cellules a été déterminé par le test MTT.

Les résultats exprimés en pourcentage de prolifération cellulaire pendant 24 h par rapport à une culture témoin non traitée, sont rapportés dans le tableau 4 ci dessous.

**TABLEAU 4**

| Facteur | Type de formulation | Prolifération (%) |
|---|---|---|
| PDGF | Forme pharmaceutique selon l'invention | 177,6 ± 3,1 |
| TGF-β | Forme pharmaceutique selon l'invention | 193,2 ± 3,7 |
| Aucun | Matériau support 60/40 seul | 102,4 ± 5,6 |

Les résultats montrent que comme dans le cas du FGF-2, les deux facteurs de croissance PDGF et TGF-β présentent une activité stimulatrice de la prolifération cellulaire. Le matériau support seul (sans facteurs de croissance) ne présente aucun effet sur les cellules.

### Exemple 6: Préparation et étude de l'activité d'une forme pharmaceutique contenant le facteur de croissance NGF

Le NGF stimule la différenciation des cellules neuronales comme les cellules de phéochromocytome de rat PC12 ou les neuroblastomes Neuro2A à des doses similaires à celles du FGF-2 (entre 20 et 100 ng/ml). Ces cellules arrêtent de proliférer et étendent des extensions neuritiques, ce qui constitue un critère morphologique indicatif de leur différenciation.

Le NGF a été formulé au sein du matériau support contenant, en poids 60 % d'oligomère et 40 % de polymère de méthylidène malonate préparés à partir du monomère 1-éthoxycarbonyl-1-éthoxycarbonylméthylènoxycarbonyl éthène (1µg de NGF formulé pour 40 mg de matériau, comme décrit dans l'exemple 2). La forme pharmaceutique selon l'invention contenant ou non le NGF a alors été mise en contact avec les cellules neuronales PC12 et Neuro2A suivant le protocole décrit dans l'Exemple 1. L'effet de stimulation de la différenciation des cellules a été quantifié par la détermination (comptage dans une grille standardisée) du pourcentage de cellules présentant des extensions neuritiques de longueur supérieure ou égale à 1 diamètre cellulaire.

Les résultats exprimés en pourcentage de cellules présentant des neurites par rapport à une culture non traitée sont rapportés dans le tableau 5 ci-dessous.

**TABLEAU 5**

| | Cellules PC12 à neurites (% du contrôle) | Cellules Neuro2A à neurites (% du contrôle) |
|---|---|---|
| Forme pharmaceutique contenant du NGF | 253,3 ± 7,2 | 181,5 ± 3,1 |
| NGF libre | 290 ± 6,3 | 200,4 ± 4,5 |
| Matériau support 60/40 | 113,2 ± 5,1 | 96,8 ± 3,1 |

Les résultats montrent que le NGF au sein de la forme pharmaceutique selon l'invention présente une activité biologique de différenciation neuronale. Le matériau support seul (sans NGF) ne présente aucun effet sur les cellules neuronales. La forme pharmaceutique avec NGF représente donc un moyen de stimuler la réparation neuronale.

### Exemple 7 : Préparation et étude de l'activité d'une forme pharmaceutique contenant de la sphingosylphosphorylcholine (SPC) sur les cellules fibroblastiques

Le métabolite sphingosylphosphorylcholine (SPC) définit une nouvelle classe de seconds messagers intracellulaires présentant un spectre large d'activité biologique de régulation de la croissance cellulaire et de la transduction du signal. Les données actuelles de la littérature sur la SPC montrent qu'elle stimule la prolifération de nombreux types cellulaires (fibroblastes, préadipocytes L1, gliomes C6, astrocytes humains...) quiescents ou en croissance exponentielle.

La SPC a été formulée au sein d'un matériau support contenant, en poids, 60 % d'oligomère et 40 % de polymère de méthylidène malonate préparés à partir du monomère 1-éthoxycarbonyl-1-éthoxycarbonylméthylènoxycarbonyl éthène (100 µg de SPC formulés pour 40 mg de matériau, comme décrit dans l'exemple 2). La forme pharmaceutique selon l'invention contenant la SPC ou son solvant contrôle a alors été mise en contact avec les cellules fibroblastiques BP-A31 suivant le protocole décrit dans l'exemple 1. L'effet d'un ajout de 3,1 % dans le puit, générant une concentration théorique finale de SPC de 15 µM, est présenté ici. La stimulation de la prolifération des cellules a été quantifié par le test MTT. Les résultats exprimés en pourcentage de prolifération cellulaire pendant 24 h par rapport à une culture témoin non traitée, sont rapportés dans le tableau 6 ci-dessous.

**TABLEAU 6**

| | Prolifération (%) |
|---|---|
| Forme pharmaceutique contenant la SPC | 186,9 ± 9 |
| SPC libre | 158,8 ± 3,2 |
| Matériau support 60/40 | 102,9± 9,7 |

Les résultats montrent que la forme pharmaceutique selon l'invention contenant de la SPC présente une activité biologique de stimulation de la prolifération des cellules BP-A31. Celle-ci est comparable à l'activité mitogénique de la SPC libre.

## Revendications

1. Forme pharmaceutique comprenant un matériau support et au moins un facteur de régulation cellulaire et/ou un facteur promoteur de prolifération cellulaire, **caractérisé en ce que** le matériau support est constitué, pour au moins 90 % en poids et de préférence pour au moins 95 % en poids, d'une composition à base de méthylidène malonate contenant :
- de 0 à 10 % en poids d'un ou plusieurs méthylidène malonate de formule (I) 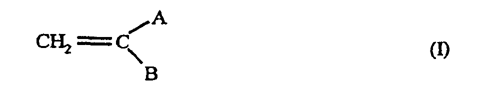
dans laquelle :
- A et B représentent indépendamment un groupe (a) ou (b) : 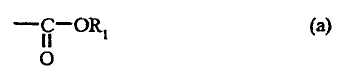 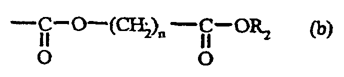
dans lesquels R₁ et R₂ représentent indépendamment un groupe alkyle linéaire ou ramifié ayant de 1 à 6 atomes de carbone, et n est un nombre entier compris entre 1 et 5 ;
- de 10 à 90 %, de préférence 50 à 90 % en poids d'un ou plusieurs oligomère(s) de méthylidène malonate présentant un poids moléculaire inférieur ou égal à 6 000 et constitué(s) d'unités récurrentes de formule (II) : 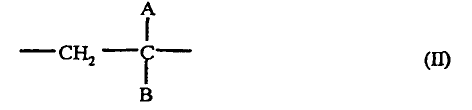
dans laquelle A et B sont tels que définis précédemment ;
- de 10 à 90 %, de préférence 10 à 50 % en poids d'un ou plusieurs polymère(s) de méthylidène malonate présentant un poids moléculaire supérieur à 6 000 et constitué(s) d'unités récurrentes de formule (II).

2. Forme pharmaceutique selon la revendication 1, **caractérisée en ce que** dans les formules (I) et (II) précitées :
- A représente un groupe (a) dans lequel R₁ représente un groupe éthyle ; et
- B représente un groupe (b) dans lequel R₂ représente un groupe éthyle et n est un nombre égal à 1.

3. Forme pharmaceutique selon les revendications 1 ou 2, **caractérisée en ce que** le matériau support est constitué pour au moins 90%, et de préférence pour au moins 95% en poids d'une composition à base de méthylidène malonate contenant :
- de 50 à 90 % en poids d'un ou plusieurs oligomère(s) de méthylidène malonate présentant un poids moléculaire inférieur ou égal à 6 000, de préférence inférieur ou égal à 3 000 et constitué(s) d'unités récurrentes de formule (II),
- de 10 à 50 % en poids d'un ou plusieurs polymères de méthylidène malonate présentant un poids moléculaire supérieur à 6 000.

4. Forme pharmaceutique selon la revendication 3, **caractérisée en ce que** la composition à base de méthylidène malonate précitée contient :
- de 55 à 65 % en poids d'un ou plusieurs oligomère(s) de méthylidène malonate présentant un poids moléculaire inférieur ou égal à 3 000 et constitué(s) d'unités récurrentes de formule (II),
- de 35 à 45 % en poids d'un ou plusieurs polymères de méthylidène malonate présentant un poids moléculaire supérieur à 6 000, de préférence supérieur à 9 000.

5. Forme pharmaceutique selon la revendication 4, **caractérisée en ce que** la composition à base de méthylidène malonate précitée contient :
- de 55 à 65 % en poids d'un ou plusieurs oligomère(s) de méthylidène malonate présentant un poids moléculaire compris entre 300 et 1 000 et constitué(s) d'unités récurrentes de formule (II),
- de 35 à 45 % en poids d'un ou plusieurs polymères de méthylidène malonate présentant un poids moléculaire compris entre 12 000 et 25 000.

6. Forme pharmaceutique selon les revendications 1 ou 2, **caractérisée en ce que** le matériau support est constitué pour au moins 90%, et de préférence pour au moins 95% en poids d'une composition à base de méthylidène malonate contenant :
- de 40 à 80 %, de préférence de 40 à 60 % en poids d'un ou plusieurs oligomère(s) de méthylidène malonate présentant un poids moléculaire inférieur ou égal à 6 000, de préférence inférieur ou égal à 3 000 et constitué(s) d'unités récurrentes de formule (II),
- de 20 à 60 %, de préférence de 40 à 60 % en poids d'un ou plusieurs polymères de méthylidène malonate présentant un poids moléculaire supérieur à 6000.

7. Forme pharmaceutique selon l'une quelconque des revendications 1 à 6, **caractérisée en ce que** le matériau support comprend jusqu'à 10% en poids d'un
ou plusieurs constituant(s) autres que la composition à base de méthylidène malonate précitée sous forme d'unités monomériques associées à des unités méthylidène malonate de formule (II), pour former des copolymères statistiques, multiblocs ou greffés..

8. Forme pharmaceutique selon la revendication 7, **caractérisée en ce que** lesdits copolymères sont constitués, pour au moins 50% de leurs unités monomériques, par des unités méthylidène malonate.

9. Forme pharmaceutique selon l'une quelconque des revendications 1 à 6, **caractérisée en ce que** le matériau support comprend jusqu'à 10% en poids d'un
ou plusieurs constituant(s) autres que la composition à base de méthylidène malonate précitée sous forme d'unités monomériques enchaînées pour former des homopolymères ou des copolymères statistiques, multiblocs ou greffés dépourvus d'unités méthylidène malonate de formule (II).

10. Forme pharmaceutique selon l'une quelconque des revendications 1 à 9, **caractérisée en ce que** le facteur de régulation cellulaire est un facteur de croissance naturel, synthétique ou recombinant.

11. Forme pharmaceutique selon l'une quelconque des revendications 1 à 10 **caractérisée en ce que** le facteur de croissance est choisi parmi les facteurs de croissance des fibroblastes (FGF), le « Platelet-Derived Growth Factor » (PDGF), le « Transforming Growth Factor β » (TGF β), le « Nerve Growth Factor » (NGF)
ou une association de plusieurs d'entre eux.

12. Forme pharmaceutique selon l'une quelconque des revendications 1 à 11, **caractérisée en ce que** le ou les facteur(s) de croissance est (sont) utilisé(s) seul(s) ou en complexe avec un ligand.

13. Forme pharmaceutique selon l'une quelconque des revendications 1 à 12, **caractérisée en ce que** ledit facteur de croissance est le facteur de croissance des fibroblastes basique ou FGF-2 et le ligand est l'héparane sulfate ou un protéoglycane d'héparane sulfate.

14. Forme pharmaceutique selon la revendication 13, **caractérisée en ce que** le facteur de croissance FGF-2 et l'héparane sulfate sont complexés dans un rapport molaire de 1 : 1 à 1 :20, en particulier de 1 : 1,5 à 1 : 10.

15. Forme pharmaceutique selon la revendication 13, **caractérisée en ce que** le facteur de croissance FGF-2 et le protéoglycane d'héparane sulfate sont complexés dans un rapport molaire de 1 : 0,01 à 1 : 0,5.

16. Forme pharmaceutique selon l'une quelconque des revendications 1 à 15, **caractérisée en ce qu'**elle comprend un promoteur de prolifération cellulaire naturel, synthétique ou recombinant.

17. Forme pharmaceutique selon la revendication 16, **caractérisée en ce que** le promoteur de prolifération cellulaire est la sphingosylphosphorylcholine.

18. Composition pharmaceutique contenant une forme pharmaceutique selon l'une quelconque des revendications 1 à 17 et un excipient pharmaceutiquement acceptable.

19. Utilisation d'une forme pharmaceutique selon l'une quelconque des revendications 1 à 17 pour la fabrication d'un implant.

20. Utilisation d'une forme pharmaceutique selon l'une quelconque des revendications 1 à 17 pour la préparation d'une composition destinée à être placée au contact d'une plaie.

21. Utilisation d'une forme pharmaceutique selon l'une quelconque des revendications 1 à 17 pour la préparation d'un médicament pour le traitement des lésions cutanées.

22. Utilisation selon la revendication 21 pour la préparation d'un médicament pour le traitement des brûlures, en particulier les brûlures des second et troisième degrés, des ulcères, des plaies dermiques et dermoépidermiques, des escarres, des ulcères cutanés ou variqueux.

23. Utilisation d'une forme pharmaceutique selon l'une quelconque des revendications 1 à 17 pour la préparation d'un médicament pour la régénération des tissus nerveux, neuromusculaires ou neuroépithéliaux.

## Claims

1. Pharmaceutical form comprising a support material and at least one cell regulation factor and/or cell proliferation promoter, **characterized in that** at least 90% by weight and preferably at least 95% by weight of the support material consists of a composition based on methylidene malonate containing:
- from 0 to 10% by weight of one or more methylidene malonates of formula (I): 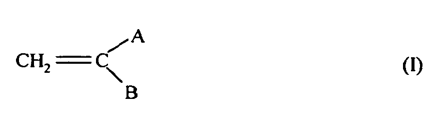
in which:
- A and B independently are a group (a) or (b) : 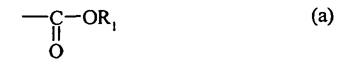 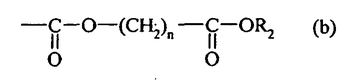
in which R₁ and R₂ independently are a linear or branched alkyl group having from 1 to 6 carbon atoms and n is an integer between 1 and 5;
- from 10 to 90% and preferably 50 to 90% by weight of one or more methylidene malonate oligomers having a molecular weight below or equal to 6000 and consisting of repeat units of formula (II): 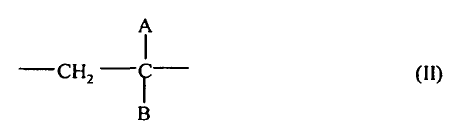
in which A and B are as defined above; and
- from 10 to 90% and preferably 10 to 50% by weight of one or more methylidene malonate polymers having a molecular weight above 6000 and consisting of repeat units of formula (II).

2. Pharmaceutical form according to claim 1, **characterized in that**, in formulae (I) and (II) given above:
- A is a group (a) in which R₁ is an ethyl group; and
- B is a group (b) in which R₂ is an ethyl group and n is 1.

3. Pharmaceutical form according to claim 1 or 2, **characterized in that** at least 90% and preferably at least 95% by weight of the support material consists of a composition based on methylidene malonate containing:
- from 50 to 90% by weight of one or more methylidene malonate oligomers having a molecular weight below or equal to 6000, preferably below or equal to 3000, and consisting of repeat units of formula (II); and
- from 10 to 50% by weight of one or more methylidene malonate polymers having a molecular weight above 6000.

4. Pharmaceutical form according to claim 3, **characterized in that** the aforementioned composition based on methylidene malonate contains:
- from 55 to 65% by weight of one or more methylidene malonate oligomers having a molecular weight below or equal to 3000 and consisting of repeat units of formula (II); and
- from 35 to 45% by weight of one or more methylidene malonate polymers having a molecular weight above 6000 and preferably above 9000.

5. Pharmaceutical form according to claim 4, **characterized in that** the aforementioned composition based on methylidene malonate contains:
- from 55 to 65% by weight of one or more methylidene malonate oligomers having a molecular weight of between 300 and 1000 and consisting of repeat units of formula (II); and
- from 35 to 45% by weight of one or more methylidene malonate polymers having a molecular weight of between 12,000 and 25,000.

6. Pharmaceutical form according to claim 1 or 2, **characterized in that** at least 90% and preferably at least 95% by weight of the support material consists of a composition based on methylidene malonate containing:
- from 40 to 80% and preferably from 40 to 60% by weight of one or more methylidene malonate oligomers having a molecular weight below or equal to 6000, preferably below or equal to 3000, and consisting of repeat units of formula (II); and
- from 20 to 60% and preferably from 40 to 60% by weight of one or more methylidene malonate polymers having a molecular weight above 6000.

7. Pharmaceutical form according to any one of claims 1 to 6, **characterized in that** the support material comprises up to 10% by weight of one or more constituents other than the aforementioned composition based on methylidene malonate, in the form of monomeric units associated with methylidene malonate units of formula (II) to form random, multiblock or graft copolymers.

8. Pharmaceutical form according to claim 7, **characterized in that** at least 50% of the monomeric units of said copolymers consist of methylidene malonate units.

9. Pharmaceutical form according to any one of claims 1 to 6, **characterized in that** the support material comprises up to 10% by weight of one or more constituents other than the aforementioned composition based on methylidene malonate, in the form of monomeric units linked to form homopolymers or random, multiblock or graft copolymers devoid of methylidene malonate units of formula (II).

10. Pharmaceutical form according to any one of claims 1 to 9, **characterized in that** the cell regulation factor is a natural, synthetic or recombinant growth factor.

11. Pharmaceutical form according to any one of claims 1 to 10, **characterized**
**in that** the growth factor is selected from fibroblast growth factors (FGFs), platelet-derived growth factor (PDGF), transforming growth factor β (TGF-β), nerve growth factor (NGF) or an association of several of these.

12. Pharmaceutical form according to any one of claims 1 to 11, **characterized**
**in that** the growth factor(s) is (are) used on its (their) own or as a complex with a ligand.

13. Pharmaceutical form according to any one of claims 1 to 12, **characterized in that** said growth factor is the basic fibroblast growth factor, or FGF-2, and the ligand is heparan sulfate or a heparan sulfate proteoglycan.

14. Pharmaceutical form according to claim 13, **characterized in that** the growth factor FGF-2 and the heparan sulfate are complexed in a molar ratio of 1:1 to 1:20 and particularly of 1:1.5 to 1:10.

15. Pharmaceutical form according to claim 13, **characterized in that** the growth factor FGF-2 and the heparan sulfate proteoglycan are complexed in a molar ratio of 1:0.01 to 1:0.5.

16. Pharmaceutical form according to any one of claims 1 to 15, **characterized**
**in that** it comprises a natural, synthetic or recombinant cell proliferation promoter.

17. Pharmaceutical form according to claim 16, **characterized in that** the cell proliferation promoter is sphingosylphosphorylcholine.

18. Pharmaceutical composition containing a pharmaceutical form according to any one of claims 1 to 17 and a pharmaceutically acceptable excipient.

19. Use of a pharmaceutical form according to any one of claims 1 to 17 for the manufacture of an implant.

20. Use of a pharmaceutical form according to any one of claims 1 to 17 for the preparation of a composition to be placed in contact with a wound.

21. Use of a pharmaceutical form according to any one of claims 1 to 17 for the preparation of a medicament for the treatment of skin lesions.

22. Use according to claim 21 for the preparation of a medicament for the treatment of bums, particularly second and third degree bums, ulcers, dermal and dermo-epidermal wounds, bedsores, skin ulcers or varicose ulcers.

23. Use of a pharmaceutical form according to any one of claims 1 to 17 for the preparation of a medicament for the regeneration of nervous, neuromuscular or neuroepithelial tissues.

## Patentansprüche

1. Pharmazeutische Form, umfassend ein Trägermaterial und mindestens einen Faktor zur Zellregulation und/oder einen Zellproliferationspromotorfaktor, **dadurch gekennzeichnet, dass** das Trägermaterial zu mindestens 90 Gew.-% und vorzugsweise zu mindestens 95 Gew.-% aus einer Zusammensetzung auf der Basis von Methylidenmalonat zusammengesetzt ist, die enthält:
- 0 bis 10 Gew.-% von einem oder mehreren Methylidenmalonat(en) der Formel (I) 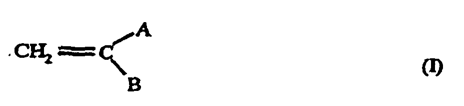
worin:
- A und B unabhängig voneinander für eine Gruppe (a) oder (b) stehen: 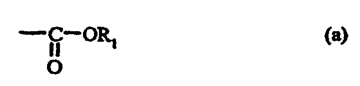 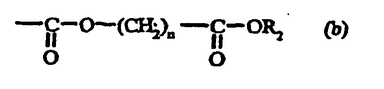
worin R₁ und R₂ unabhängig voneinander für eine lineare oder verzweigte Alkylgruppe mit 1 bis 6 Kohlenstoffatomen stehen, und n für eine ganze Zahl zwischen 1 bis 5 steht;
- 10 bis 90 Gew.-%, vorzugsweise 50 bis 90 Gew.-%, von einem oder mehreren Oligomer(en) von Methylidenmalonat mit einem Molekulargewicht von weniger als oder gleich 6000 und zusammengesetzt aus Wiederholungseinheiten der Formel (II): 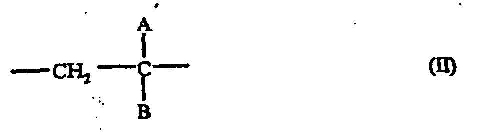
worin A und B wie vorstehend definiert sind;
- 10 bis 90 Gew.-%, vorzugsweise 10 bis 50 Gew.-%, von einem oder mehreren Polymer(en) von Methylidenmalonat mit einem Molekulargewicht von mehr als 6000 und zusammengesetzt aus Wiederholungseinheiten der Formel (II).

2. Pharmazeutische Form gemäß Anspruch 1, **dadurch gekennzeichnet, dass** in den vorstehend angegebenen Formeln (I) und (II):
- A für eine Gruppe (a) steht, worin R₁ für eine Ethylgruppe steht; und
- B für eine Gruppe (b) steht, worin R₂ für eine Ethylgruppe steht und n für eine Zahl von gleich 1 steht.

3. Pharmazeutische Form gemäß den Ansprüchen 1 oder 2, **dadurch gekennzeichnet, dass** das Trägermaterial zu mindestens 90 Gew.-%, und vorzugsweise zu mindestens 95 Gew.-%, aus einer Zusammensetzung auf der Basis von Methylidenmalonat zusammengesetzt ist, enthaltend:
- 50 bis 90 Gew.-% von einem oder mehreren Oligomer(en) von Methylidenmalonat mit einem Molekulargewicht von weniger als oder gleich 6000, vorzugsweise von weniger als oder gleich 3000 und zusammengesetzt aus Wiederholungseinheiten der Formel (II),
- 10 bis 50 Gew.-% von einem oder mehreren Polymeren von Methylidenmalonat mit einem Molekulargewicht von mehr als 6000.

4. Pharmazeutische Form gemäß Anspruch 3, **dadurch gekennzeichnet, dass** die vorstehend angegebene Zusammensetzung auf der Basis von Methylidenmalonat enthält:
- 55 bis 65 Gew.-% von einem oder mehreren Oligomer(en) von Methylidenmalonat mit einem Molekulargewicht von weniger als oder gleich 3000 und zusammengesetzt aus Wiederholungseinheiten der Formel (II),
- 35 bis 45 Gew.-% von einem oder mehreren Polymeren von Methylidenmalonat mit einem Molekulargewicht von mehr als 6000, vorzugsweise mehr als 9000.

5. Pharmazeutische Form gemäß Anspruch 4, **dadurch gekennzeichnet, dass** die vorstehend angegebene Zusammensetzung auf der Basis von Methylidenmalonat enthält:
- 55 bis 65 Gew.-% von einem oder mehreren Oligomer(en) von Methylidenmalonat mit einem Molekulargewicht zwischen 300 und 1000 und zusammengesetzt aus Wiederholungseinheiten der Formel (II),
- 35 bis 45 Gew.-% von einem oder mehreren Polymeren von Methylidenmalonat mit einem Molekulargewicht zwischen 12.000 und 25.000.

6. Pharmazeutische Form gemäß den Ansprüchen 1 oder 2, **dadurch gekennzeichnet, dass** das Trägermaterial zu mindestens 90 Gew.-%, und vorzugsweise zu mindestens 95 Gew.-%, aus einer Zusammensetzung auf der Basis von Methylidenmalonat zusammengesetzt ist, die enthält:
- 40 bis 80 Gew.-%, vorzugsweise 40 bis 60 Gew.-%, von einem oder mehreren Oligomer(en) von Methylidenmalonat mit einem Molekulargewicht von kleiner als oder gleich 6000, vorzugsweise kleiner als oder gleich 3000, und zusammengesetzt aus Wiederholungseinheiten der Formel (II),
- 20 bis 60 Gew.-%, vorzugsweise 40 bis 60 Gew.-% von einem oder mehreren Polymeren von Methylidenmalonat mit einem Molekulargewicht von mehr als 6000.

7. Pharmazeutische Form gemäß einem der Ansprüche 1 bis 6, **dadurch gekennzeichnet, dass** das Trägermaterial bis zu 10 Gew.-% eines oder mehrerer Bestandteil(e), der bzw. die von der vorstehend beschriebenen Zusammensetzung auf der Basis von Methylidenmalonat verschieden ist bzw. sind, in Form von monomeren Einheiten, die mit Methylidenmalonateinheiten der Formel (II) verbunden sind, enthält, um statistische, Multiblock- oder gepfropfte Copolymere zu bilden.

8. Pharmazeutische Form gemäß Anspruch 7, **dadurch gekennzeichnet, dass** die Copolymere zu mindestens 50 % ihrer monomeren Einheiten aus Methylidenmalonateinheiten zusammengesetzt sind.

9. Pharmazeutische Form gemäß einem der Ansprüche 1 bis 6, **dadurch gekennzeichnet, dass** das Trägermaterial bis zu 10 Gew.-% eines oder mehrerer Bestandteil(e), der bzw. die von der vorstehend beschriebenen Zusammensetzung auf der Basis von Methylidenmalonat verschieden ist bzw. sind, in Form von verknüpften Monomereinheiten enthält, um Homopolymere oder statistische, Multiblock- oder gepfropfte Copolymere zu bilden, die frei sind von Methylidenmalonateinheiten der Formel (II).

10. Pharmazeutische Form gemäß einem der Ansprüche 1 bis 9, **dadurch gekennzeichnet, dass** der Faktor zur Zellregulation ein natürlicher, synthetischer oder rekombinanter Wachstumsfaktor ist.

11. Pharmazeutische Form gemäß einem der Ansprüche 1 bis 10, **dadurch gekennzeichnet, dass** der Wachstumsfaktor ausgewählt ist aus den Fibroblastenwachstumfaktoren (FGF), dem "Platelet-Derived Growth Factor" (PDGF), dem "Transforming Growth Factor β" (TGF β), dem "Nerve Growth Factor" (NGF) oder einer Assoziation von mehreren dieser Faktoren.

12. Pharmazeutische Form gemäß einem der Ansprüche 1 bis 11, **dadurch gekennzeichnet, dass** der oder die Wachstumsfaktor(en) einzeln oder als Komplex mit einem Liganden verwendet wird bzw. werden.

13. Pharmazeutische Form gemäß einem der Ansprüche 1 bis 12, **dadurch gekennzeichnet, dass** es sich bei dem Wachstumsfaktor um den basischen Fibroblastenwachstumsfaktor oder FGF-2 und bei dem Liganden um Heparansulfat oder ein Heparansulfat-Proteoglycan handelt.

14. Pharmazeutische Form gemäß Anspruch 13, **dadurch gekennzeichnet, dass** der Wachstumsfaktor FGF-2 und Heparansulfat in einem Molverhältnis von 1 : 1 bis 1 : 20, insbesondere von 1 : 1,5 bis 1 : 10, komplexiert sind.

15. Pharmazeutische Form gemäß Anspruch 13, **dadurch gekennzeichnet, dass** der Wachstumsfaktor FGF-2 und Heparansulfat-Proteoglycan in einem Molverhältnis von 1 : 0,01 bis 1 : 0,5 komplexiert sind.

16. Pharmazeutische Form gemäß einem der Ansprüche 1 bis 15, **dadurch gekennzeichnet, dass** sie einen natürlichen, synthetischen oder rekombinanten Zellproliferationspromotor umfasst.

17. Pharmazeutische Form gemäß Anspruch 16, **dadurch gekennzeichnet, dass** es sich bei dem Zellproliferationspromotor um Sphingosylphosphorylcholin handelt.

18. Pharmazeutische Zubereitung, umfassend eine pharmazeutische Form gemäß einem der Ansprüche 1 bis 17 und einen pharmazeutisch annehmbaren Hilfsstoff.

19. Verwendung einer pharmazeutischen Form gemäß einem der Ansprüche 1 bis 17 zur Herstellung eines Implantats.

20. Verwendung einer pharmazeutischen Form gemäß einem der Ansprüche 1 bis 17 zur Herstellung einer Zubereitung, die für eine Auftragung in Kontakt mit einer Wunde vorgesehen ist.

21. Verwendung einer pharmazeutischen Form gemäß einem der Ansprüche 1 bis 17 zur Herstellung eines Medikaments zur Behandlung von Hautverletzungen.

22. Verwendung gemäß Anspruch 21 zur Herstellung eines Medikaments zur Behandlung von Verbrennungen, insbesondere Verbrennungen zweiten oder dritten Grades, Geschwüren, Hautwunden und dermoepidermischen Wunden, Wundliegen, Hautgeschwüren oder varikösen Geschwüren.

23. Verwendung einer pharmazeutischen Form gemäß einem der Ansprüche 1 bis 17 zur Herstellung eines Medikaments zur Regeneration von Nervengewebe, neuromuskulärem Gewebe oder Neuroepithelgewebe.
